# EUROPEAN PATENT SPECIFICATION

(11) **EP 1 308 178 B1**
(45) Date of publication and mention of the grant of the patent: **30.03.2005**
(21) Application number: 02017192.2
(22) Date of filing: 31.07.2002
(51) Int. Cl.: A61M 16/12

(54) **Dosing system**
Dosierungssytem
Système de dosage

(30) Priority: 04.10.2001 SE 0103309
(43) Date of publication of application: 07.05.2003
(73) Proprietor: Maquet Critical Care AB, 171 95 Solna (SE)
(72) Inventor: Rydgren, Göran, 23044 Bunkeflostrand (SE)
(74) Representative: Stein, Jan Anders Lennart

(56) References cited:
- EP-A- 0 640 356
- EP-A- 0 640 357
- WO-A-98/31282
- US-A- 5 839 433

## Description

The present invention relates to a dosing system for supplying an additive to an inspiration gas, in particular to a dosing system in which the timing of the delivery of the dose within an inspiration phase is controlled by monitoring the contents of the expiration gas.

It is known that the additive nitrogen monoxide (NO) is preferentially taken up in the alveoli of the lung. It is also known that the amount of NO taken up by the lung can be varied by varying the timing (that is one or more of the start time, the end time, duration and presence within an inspiration phase) of delivery of a dose of NO into an inspiration gas which is to be supplied to a patient. Thus the operation of an NO dosing unit may be controlled dependent on the level of NO detected in expiration gas so as to improve the efficiency of the dosing.

Certain additives, such as surfactants and prostacyclin, because they are effective only if they reach a certain region of the lung, are usually dosed throughout an entire inspiration phase in an attempt to ensure that they reach that lung region. This is both inefficient and costly as almost 90% of the dosed additive is typically present in expiration gas.

EP 640 357 discloses a gas mixture and device for delivering the gas mixture. The gas mixture comprises NO and an inert tracer gas in exact proportions, where the tracer gas is present in higher concentrations than NO. By measuring the concentration of tracer gas in breathing gas after dosing the gas mixture, the concentration of NO can be determined from the concentration of tracer gas and the proportionality. Since the tracer gas is inert, the concentration of NO supplied to a patient can even be determined by measuring the concentration of tracer gas in exhaled gas.

A device as defined in the preamble of claim 1 is disclosed in WO 98/31282. Particularly WO 98/31282 discloses a gas-supply system where one or several gases can be added to respiration gas at varying proportions by means of a control device.

According to a first aspect of the present invention there is provided a dosing system as described in and characterised by the present Claim 1. Thus by monitoring the expiration gas for components related to the indicator for example changes in level of the component consequent on different introduction times of the indicator, the timing of delivery of a dose of an additive into an inspiration gas may be adjusted. A reduced amount of additive may then be delivered into the respiratory system of the patient.

Usefully, the indicator material is selected so as to be preferentially taken up within a known region of the respiratory system of the patient, most usefully within a region associated with a preferential action or take-up the additive. Thus the timing of the delivery of the dose may be varied to ensure a delivery of the additive to the known region.

According to a second aspect of the present invention there is provided a dosing system comprising a dosing monitor as described in and characterised by the present Claim 6.

Exemplary embodiments of the invention will now be described with reference to the drawings of the accompanying figures of which:
Fig. 1 shows a schematic of a dosing system according to the present invention operably connected to a ventilator.
Fig. 2 shows a schematic of a dosing monitor according to the present invention operably connected to a ventilator.

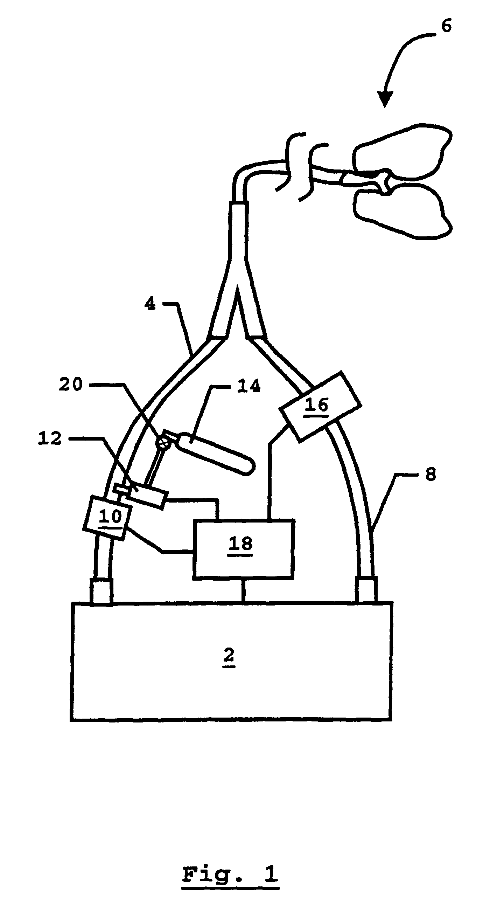

Considering now the example of a dosing system as shown in Fig. 1, a standard ventilator 2 of known operation and construction, is arranged to supply an inspiration gas through an inspiration line 4 and to the respiratory system of a patient 6 during inspiration phases of patient breathing cycles. The ventilator 2 is also arranged to receive, during expiration phases, expiration gas from the respiratory system 6 through an expiration line 8.

As shown in Fig. 1, the exemplary dosing system comprises a dosing unit 10, an injection device 12, a source of gaseous indicator material 14, a detector unit 16 and an analyser/control unit 18. The dosing unit 10, such as a conventional nebuliser or vaporiser, is connected to the inspiration line 4 and is operable to dose inspiration gas within that line 4 with a predetermined amount of an additive, such as a physiologically active additive having a dilatory or narcotic effect. The operation of the dosing unit to deliver the dose of additive is controlled by the analyser/control unit 18 which is configured to control amongst other things, the start time, stop time or dosing profile (variations in additive flow with time during the delivery) of the dosing unit.

The control unit 18 of the present embodiment is also operably connected to the ventilator 2 to receive a signal indicative of the initiation of an inspiration phase and thus the onset of supply of inspiration gas by the ventilator. It will be appreciated by those skilled in the art that a dedicated flow meter within the inspiration line 4 may alternatively be used to generate the indicative signal. This signal is employed by the analyser/control unit 18, in a known manner, as a timing signal against which the operation of the dosing unit 10 is controlled. The analyser/control unit 18 is additionally connected to control the injection device 12 to inject a known amount of indicator material into the inspiration gas flow at a variable onset time within the inspiration phase. This onset time may be readily established using a known mechanical or electronic timer initiated by the timing signal already used by the analyser/control unit 18 in the control of the dosing unit 10.

The injection device 12, which may be a on/off valve or proportional valve of known construction, is used as a means to introduce a known amount of marker material into inspiration gas, either together with or separate from the delivery of the additive, within the inspiration line 4. It will be appreciated by those skilled in the art that the dosing unit 10 itself may also be employed as the means to introduce the marker material without departing from the claimed invention. The injection device 12 is connectable to the source of indicator material 14 through the operation of the flow control valve 20.

The source 14 is here a commercially available cylinder of pressurised NO diluted in nitrogen to provide a known concentration of the NO. Other gaseous indicator material may be substituted for NO, such as Carbon Monoxide (CO) which is also preferentially taken-up in the alveoli of the respiratory system 6, or Nitrous Oxide (N₂O) which is preferentially taken-up in the bronchiole of the respiratory system. A material which is transformed into a readily detectable gas when taken-up in the respiratory system 6, for example the enzyme L-arginine is known to oxidise to produce NO as it penetrates cell membranes of the respiratory system 6, may also be usefully employed as the indicator material.

The detector unit 16 is arranged to detect levels of a component of expiration gas passed from the respiratory system of the patient 6 which is related to the indicator material. As can be seen from the examples of indicator material provided above, dependent on the indicator material selected the component may be the indicator material itself or something which results from an interaction of the indicator material with the respiratory system 6. As illustrated in the embodiment of Fig.1, the detector unit 16 may be conveniently connected to the expiration line 8 and is configured to provide an output indicative of the detected level to the analyser/control unit 18.

In one mode of operation of the dosing system illustrated by Fig.1 the dosing unit 10 is supplied with a medicament, such as a surfactant, which is effective only if taken-up in the alveoli of the respiration system 6. The analyser/control unit 18 is provided with operating instructions, such as in the form of program code portions when the analyser/control unit 18 is microprocessor based, to control the injection device 12 to introduce NO at a different onset time in each of a plurality of inspiration phases and to store a value representing the monitored level of the component of expiration gas in expiration phases consequent on each of the inspiration phases, as obtained from the detection unit 16. The analyser/control unit 18 then operates to analyse the stored values to determine an onset time at which the take up of NO in the respiratory system is greatest. Since, in this example, it is desired to ensure that the medicament is taken up in the alveoli then the analyser/control unit 18 sets the start time for delivery of medicament by the dosing unit 10 to be the determined onset time for NO.

If the additive delivered by the dosing unit 10 is intended for take-up in another region of the respiratory system 6, such as, for example, in the bronchial tree for asthma medicine, then using NO as the marker material and operating the system as described above to obtain the onset time for maximum NO take-up the analyser/control unit 18 may be arranged to set a start time for delivery time shifted (here delayed) by a predetermined amount dependent on the separation between the take-up region of the indicator material and the target delivery region of the additive. It will be appreciated that the analyser/control unit 18 may be programmed with a plurality of pre-set time shifts which may be automatically selected dependent on user input indicator take-up and additive target regions of the respiratory system 6 (perhaps input as indicator material and additive). Alternatively, in this case the indicator source 14 may be replaced with an indicator which is also preferably taken-up in the bronchial tree, for example N₂O, a suitable detector provided in the detector unit 16 and the analyser/control unit 18 configured to operate the dosing device 10 to start the delivery of the additive at a time equal to an onset time determined for greatest take-up of the indicator.

Considering now a different mode of operation of the system according to Fig. 1. In this mode the dosing device 10 is operated by the analyser/control unit 18 in dependence of an efficacy of the medicament in changing the physical properties of the respiratory system 6, as indicated by the level of component in expiration gas monitored by the detector unit 16. In the present example the source of indicator 14 is selected to indicate a preferential take-up in a different part of the respiratory system 6 to that of the additive delivered by the dosing unit 10 and in particular the region of take-up indicated by the indicator material is after that of the additive in the direction of inspiration gas flow from the ventilator 2. To illustrate the mode of operation it is assumed that the additive is selected to provide bronchial spasm relaxation, such as a cortison based medicament like pulmicort™. The indicator gas in the source 14 is CO, or any other material which preferentially interacts with the alveoli, and is preferably introduced into inspiration gas within the inspiration line within the same inspiration phase as the additive is delivered by the dosing unit 10. The detector unit 16 is arranged to monitor levels of CO in expiration gas within the expiration line 8 and to provide an output to the analyser/control unit 18 indicative of the same. The analyser/control unit 18 is programmed to compare a difference between a level of CO introduced and a monitored level within the same breathing cycle with a reference level and to inhibit delivery of the additive in subsequent inspiration phases when the comparison indicates a take-up of the CO in the alveoli. This shows that the additive has been effective in removing the spasm and is no longer required. The analyser/control unit 18 is further arranged to and re-initiate delivery by the dosing unit 10 when the comparison indicates that take-up of CO is being reduced.

Additionally, the source 14 may comprise a second indicator material, such as N₂O, selected to interact preferentially with the bronchiole of the respiratory system 6 and the detector unit 16 configured to also monitor levels of this gas within the expiration gas. The analyser/control unit 18 is configured to operate as described for the previous mode to determine an onset time at which the interaction of the indicator N₂O is greatest and further to track this determined time with the number of inspiration phases within which the additive is delivered. This provides information on the position and duration of the spasm region within the bronchiole and may be used to vary the start time of delivery of the additive to match the movement of the spasm through the bronchiole.

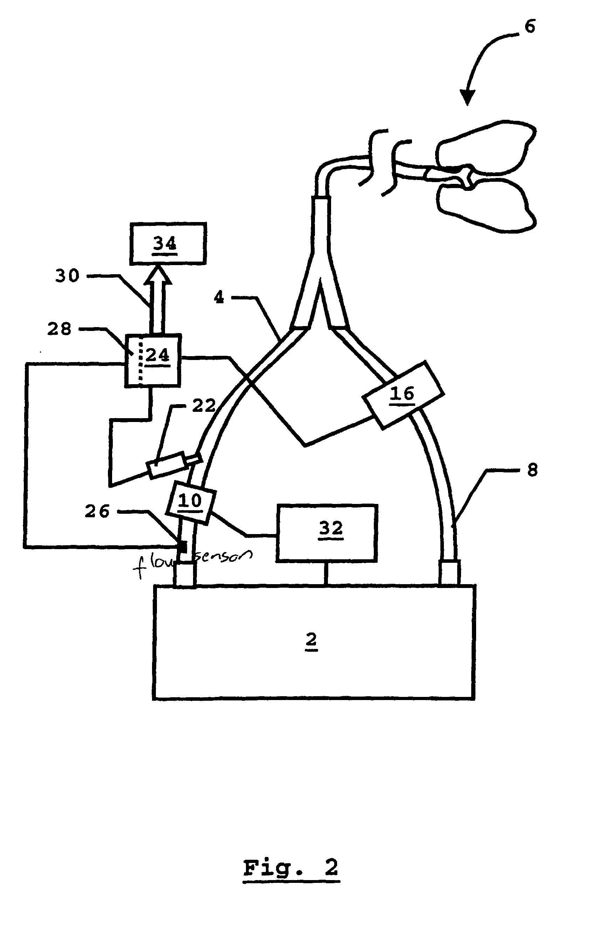

Considering now the dosing monitor according to the present invention which is illustrated in the exemplary embodiment of Fig. 2. In this figure items which are common to both the embodiment of Fig. 1 and the one of Fig. 2 are provided with identical reference numerals. A dosing monitor comprises a means for introducing an indicator material 22, which may be the same as the injection device 12 of Fig. 1; a detector unit 16 for detecting components of expiration gas related to indicator material introduced into inspiration gas by the means 22; an analyser/control unit 24 and a flow monitor 26. The analyser/control unit 24 is arranged to receive a signal from the flow monitor 26 which is positioned, in use, in the inspiration line 4 connected to the ventilator 2, indicating a start of an inspiration phase. Such flow monitors are well known in the art of mechanical ventilation and may be configured to monitor one or both of flow rate and pressure within the inspiration line 4. It will be appreciated by those skilled in the art that the flow monitor 26 allows operation of the dosing monitor independent of the make and type of the ventilator 2.

The signal from the flow monitor 26 is employed by the analyser/control unit 24 as a timing signal to start a timing device 28 which forms a part of the analyser unit (in a similar manner to the use of timing signal from the ventilator 2 by the analyser/control unit of the embodiment of Fig. 1). After a predetermined time has elapsed a signal from the timing device 28 causes the analyser/control unit 24 to trigger the means 22 to introduce an amount of indicator material into the inspiration line 4. The detector unit 16 operates to provide an output to the analyser/control unit 24 indicative of the monitored component level.

The analyser/control unit 24 is configured to operate in a manner substantially similar to those described with respect to the various modes of operation of the analyser/control unit 18 of Fig. 1 to provide an output signal 30 related to the dosing of inspiration gas within the inspiration line 4 by the dosing device 10 in a known manner, under control of a control unit 32. This output signal 30 may be provided by the analyser/control unit 24 in a form suitable to drive a display device to provide a visual indication of, for example, delivery timing parameters (such as start time, end time or duration) for input into the control unit 32 to vary the operation of the dosing device, or of the efficacy of the additive dosed by the dosing device 10.

## Claims

1. A dosing system comprising a dosing unit (10) for delivering an amount of an additive into an inspiration gas; control (16,18) means for monitoring (16) the contents of an expiration gas and for controlling (18) the dosing unit (10) to vary a timing of delivery of the additive responsive to the monitored contents; **characterised in that**
the system includes means (12) for introducing into inspiration gas at an onset time within an inspiration phase an indicator material; and **in that**
the control means (16,18) is adapted to monitor a level of a predetermined component of the expiration gas related to the indicator material during a successive expiration phase and to control the dosing unit (10) to vary the timing of delivery of the additive dependent on both the monitored level and the onset time.

2. A dosing system as claimed in claim 1, **characterised in that**
the means (12) for introducing an indicator material is configured to vary the onset time for different inspiration phases; and **in that**
the control means (18) is adapted to control dosing unit (10) to vary the timing of delivery dependent on variations in the monitored level with onset time.

3. A dosing system as claimed in claim 2, **characterised in that**
the control means (16,18) is configured to determine from the variations in the monitored level an onset time for a maximum take-up of the additive and to control the dosing unit (10) to start delivery dependent on the determined onset time.

4. A dosing system as claimed in claim 2 or 3, **characterised in that**
the control means (16,18) is configured to determine from the variations in the monitored level one or both of an upper and a lower onset time each indicative of a different predetermined level of take-up of the additive and to control the dosing unit (10) to vary a duration of the delivery dependent on the one or both determined onset times.

5. A dosing system as claimed in any preceding claim, **characterised in that**
there is further provided for operable connection to the means (12) for introducing an indicator material a source (14) of indicator material containing one or more gases, each selected to be preferentially taken up at a different known region of the respiratory system (6) of a patient.

6. A dosing system as claimed in any preceding claim, **characterised by**
a dosing monitor having means (22) for introducing into inspiration gas during an inspiration phase a indicator material for indicating take-up of an additive within the respiratory system (6) of a patient, and a detector (16) adapted to monitor a level of a predetermined component of the expiration gas related to the indicator material during a successive expiration phase and to provide an output signal indicative of the same.

7. A dosing system as claimed in claim 6, **characterised in that**
there is further provided an analyser (24) adapted to compare the output signal of the detector (16) from each of a plurality of expiration phases, at least one of which expiration phase is successive to an inspiration phase in which inspiration gas is dosed with an additive, and to provide an output signal (30) dependent on the comparison.

8. A dosing system claimed in any preceding claim, **characterised in that**
said predetermined component of the expiration gas is the indicator material.

9. A dosing system claimed in any preceding claim, **characterised in that**
said predetermined component of the expiration gas is a material which results from an interaction of the indicator material with the respiratory system.

## Patentansprüche

1. Dosierungssystem, umfassend eine Dosierungseinheit (10) zur Abgabe einer Menge eines Zusatzstoffes in ein Inspirationsgas, Steuermittel (16, 18) zum Überwachen (16) des Inhalts eines Exspirationsgases und zum Steuern (18) der Dosierungseinheit (10), um den Zeitpunkt der Abgabe des Zusatzstoffes in Antwort auf den überwachten Inhalt zu variieren, **dadurch gekennzeichnet, dass**
das System Mittel (12) zum Einbringen eines Indikatormaterials in das Inspirationsgas zu einem Einsatzzeitpunkt innerhalb einer Inspirationsphase hat, und dass
die Steuermittel (16, 18) dazu geeignet sind, den Anteil einer vorgegebenen Komponente des Exspirationsgases, die mit dem Indikatormaterial in Zusammenhang steht, während einer nachfolgenden Exspirationsphase zu überwachen und die Dosierungseinheit (10) zu steuern, um den Zeitpunkt der Abgabe des Zusatzstoffes sowohl abhängig von dem überwachten Anteil als auch dem Einsatzzeitpunkt zu variieren.

2. Dosierungssystem nach Anspruch 1, **dadurch gekennzeichnet, dass**
die Mittel (12) zum Einbringen eines Indikatormaterials derart konfiguriert sind, dass sie den Einsatzzeitpunkt für verschiedene Inspirationsphasen variieren, und dass
die Steuermittel (18) dazu geeignet sind, die Dosierungseinheit (10) zu steuern, um den Zeitpunkt der Abgabe abhängig von Veränderungen des überwachten Anteils mit dem Einsatzzeitpunkt zu variieren.

3. Dosierungssystem nach Anspruch 2, **dadurch gekennzeichnet, dass** die Steuermittel (16, 18) derart konfiguriert sind, dass sie aus den Veränderungen des überwachten Anteils einen Einsatzzeitpunkt für eine maximale Aufnahme des Zusatzstoffes festsetzen und die Dosierungseinheit (10) steuern, um abhängig von dem festgesetzten Einsatzzeitpunkt die Abgabe zu starten.

4. Dosierungssystem nach Anspruch 2 oder 3, **dadurch gekennzeichnet, dass** die Steuermittel (16, 18) derart konfiguriert sind, dass sie aus den Veränderungen des überwachten Anteils einen oberen und/oder einen unteren Einsatzzeitpunkt festsetzen, die jeweils einen unterschiedlichen vorgegebenen Grad der Aufnahme des Zusatzstoffes anzeigen, und die Dosierungseinheit (10) steuern, um eine Dauer der Abgabe abhängig von dem einen oder von beiden festgesetzten Einsatzzeitpunkten zu variieren.

5. Dosierungssystem nach einem der vorhergehenden Ansprüche, **dadurch gekennzeichnet, dass** ferner zur betriebsfähigen Verbindung mit den Mitteln (12) zum Einbringen eines Indikatormaterials eine Quelle (14) von Indikatormaterial vorgesehen ist, die ein oder mehrere Gase enthält, die jeweils derart ausgewählt sind, dass sie vorzugsweise an einem jeweils anderen bekannten Bereich des Respirationssystems (6) eines Patienten aufgenommen werden.

6. Dosierungssystem nach einem der vorhergehenden Ansprüche, **gekennzeichnet, durch** einen Dosierungsmonitor mit Mitteln (22) zum Einbringen eines Indikatormaterials in ein Inspirationsgas während einer Inspirationsphase zur Anzeige der Aufnahme eines Zusatzstoffes innerhalb des Respirationssystems (6) eines Patienten, und einen Detektor (16), der dazu geeignet ist, den Anteil einer vorgegebenen Komponente des Exspirationsgases, die mit dem Indikatormaterial in Zusammenhang steht, während einer nachfolgenden Exspirationsphase zu überwachen und ein Ausgangssignal, das diesen anzeigt, zu liefern.

7. Dosierungssystem nach Anspruch 6, **dadurch gekennzeichnet, dass** ferner ein Analysator (24) vorgesehen ist, der dazu geeignet ist, das Ausgangssignal des Detektors (16) aus jeder einer Vielzahl von Exspirationsphasen zu vergleichen, wobei mindestens eine der Exspirationsphasen auf eine Inspirationsphase folgt, in der dem Inspirationsgas ein Zusatzstoff dosiert zugesetzt wurde, und abhängig von dem Vergleich ein Ausgangssignal (30) zu liefern.

8. Dosierungssystem nach einem der vorhergehenden Ansprüche, **dadurch gekennzeichnet, dass** die vorgegebene Komponente des Exspirationsgases das Indikatormaterial ist.

9. Dosierungssystem nach einem der vorhergehenden Ansprüche, **dadurch gekennzeichnet, dass** die vorgegebene Komponente des Exspirationsgases ein Material ist, das aus einer Wechselwirkung des Indikationsmaterials mit dem Respirationssystems resultiert.

## Revendications

1. Système de dosage comprenant une unité (10) de dosage pour envoyer une quantité d'un additif dans un gaz d'inspiration; des moyens (16, 18) de commande pour contrôler (16) les contenus d'un gaz d'expiration et pour commander (18) l'unité (10) de dosage de manière à modifier une cadence d'envoi de l'additif en réponse aux contenus contrôlés, **caractérisé en ce que**
le système comprend des moyens (12) d'introduction d'une substance indicatrice dans du gaz d'inspiration à un instant de commencement dans une phase d'inspiration; et **en ce que**
les moyens (16, 18) de commande sont conçus pour contrôler une teneur d'un constituant déterminé à l'avance du gaz d'expiration apparenté à la substance indicatrice pendant une phase d'expiration successive et pour commander l'unité (10) de dosage de manière à modifier la cadence d'envoi de l'additif en fonction à la fois de la teneur contrôlée et de l'instant de commencement.

2. Système de dosage suivant la revendication 1, **caractérisé en ce que**
les moyens (12) d'introduction d'une substance indicatrice sont configurés pour modifier l'instant de commencement pour des phases d'inspiration différentes; et **en ce que**
les moyens (18) de commande sont conçus pour commander l'unité (10) de dosage afin de modifier la cadence d'envoi en fonction des variations de la teneur contrôlée avec l'instant de commencement.

3. Système de dosage suivant la revendication 2, **caractérisé en ce que**
les moyens (16, 18) de commande sont configurés pour déterminer à partir des variations de la teneur contrôlée, un instant de commencement pour une absorption maximum de l'additif et pour commander l'unité (10) de dosage afin de faire débuter l'envoi en fonction de l'instant de commencement déterminé.

4. Système de dosage suivant la revendication 2 ou 3, **caractérisé en ce que**
les moyens (16, 18) de commande sont configurés pour déterminer à partir des variations de la teneur contrôlée l'un ou les deux d'un instant de commencement supérieur et inférieur indiquant chacun un niveau déterminé à l'avance différent d'absorption de l'additif et pour commander l'unité (10) de dosage pour modifier une durée de l'envoi en fonction de l'un ou des deux instants de commencement déterminés.

5. Système de dosage suivant l'une quelconque des revendications précédentes, **caractérisé en ce qu'**
il est prévu en outre en vue d'être reliée fonctionnellement aux moyens (12) d'introduction d'une substance indicatrice, une source (14) de substance indicatrice contenant un gaz ou plusieurs gaz sélectionné chacun pour être absorbé préférentiellement en une région connue différente du système (6) respiratoire d'un patient.

6. Système de dosage suivant l'une quelconque des revendications précédentes, **caractérisé par** un dispositif de contrôle de dosage ayant des moyens (22) d'introduction dans du gaz d'inspiration pendant une phase d'inspiration d'une substance indicatrice pour indiquer une absorption d'un additif au sein du système (6) respiratoire d'un patient, et un détecteur (16) conçu pour contrôler une teneur d'un constituant déterminé à l'avance du gaz d'expiration apparenté à la substance indicatrice pendant une phase d'expiration successive et à donner un signal de sortie l'indiquant.

7. Système de dosage suivant la revendication 6, **caractérisé en ce qu'**
il est prévu en outre un analyseur (24) conçu pour comparer le signal de sortie du détecteur (16) provenant de chacune d'une pluralité de phases d'expiration, au moins l'une des phases d'expiration succédant à une phase d'inspiration dans laquelle du gaz d'inspiration est additionné de manière dosé d'un additif, et pour fournir un signal (30) de sortie qui dépend de la comparaison.

8. Système de dosage suivant l'une quelconque des revendications précédentes, **caractérisé en ce que**
le constituant déterminé à l'avance du gaz d'expiration est la substance indicatrice.

9. Système de dosage suivant l'une quelconque des revendications précédentes, **caractérisé en ce que**
le constituant déterminé à l'avance du gaz d'expiration est une substance qui provient d'une interaction entre la substance indicatrice et le système respiratoire.
